# EUROPÄISCHE PATENTSCHRIFT

(11) **EP 2 403 392 B1**
(45) Veröffentlichungstag und Bekanntmachung des Hinweises auf die Patenterteilung: **03.01.2018**
(21) Anmeldenummer: 09795364.0
(22) Anmeldetag: 30.11.2009
(51) Int. Cl.: A61B 1/00, A61B 1/04, A61B 1/05, A61B 5/07, G06T 7/00, G06T 7/73, A61B 34/20, A61B 90/00, A61B 34/00

(54) **VORRICHTUNG ZUR NAVIGATION EINER ENDOSKOPIEKAPSEL**
DEVICE FOR NAVIGATION OF AN ENDOSCOPIC CAPSULE
DISPOSITIF POUR LA NAVIGATION D'UNE CAPSULE D'ENDOSCOPIQUE

(30) Priorität: 05.03.2009 DE 102009011831
(43) Veröffentlichungstag der Anmeldung: 11.01.2012
(73) Patentinhaber: Siemens Healthcare GmbH, 91052 Erlangen (DE)
(72) Erfinder: DEGENHARDT, Achim, 91054 Erlangen (DE); JUNGKUNZ, Clemens, 91056 Erlangen (DE); KUTH, Rainer, 91315 Höchstadt (DE)
(86) Internationale Anmeldenummer: PCT/EP2009/066028
(87) Internationale Veröffentlichungsnummer: WO 2010/099841

(56) Entgegenhaltungen:
- EP-A1- 1 862 106
- WO-A2-2006/045011
- DE-A1-102005 032 577

## Beschreibung

Die Erfindung betrifft eine Vorrichtung zur Navigation einer Endoskopiekapsel gemäß des Anspruchs 1. Bevorzugte Ausführungsformen sind in den abhängigen Ansprüchen definiert. Sämtliche in der Beschreibung und in den Zeichnungen offenbarte, jedoch nicht-beanspruchte Ausführungsformen und Verfahren bilden nicht Teil der Erfindung und sind lediglich als Beispiele zu betrachten. Eine in Rede stehende Endoskopiekapsel ist z.B. aus der DE 101 42 253 bekannt. Die vorliegende Endoskopiekapsel beinhaltet zumindest eine Kamera, die zeitaktuelle Bilder oder ein Live-Videobild sendet. Derartige Kapseln können außerdem verschiedene Inspektions-, Diagnose- oder Therapieeinrichtungen. Dies können z.B. eine Videokamera, eine Biopsiezange, ein Clip oder ein Medikamentenreservoir sein. Die Kapsel enthält weiterhin ein magnetisierbares oder permanentmagnetisches Element, mit Hilfe dessen die Kapsel im Patienten drahtlos bewegt wird. Hierzu liegt der Patient ganz oder teilweise in einem elektrischen Spulensystem aus mehreren, z.B. 14 Einzelspulen. Vom Spulensystem werden geeignete Magnetfelder bzw. Gradientenmagnetfelder erzeugt, welche an der sich im Patienten befindlichen Kapsel bzw. am magnetischen Element Kräfte bzw. Drehmomente erzeugen. So kann die Kapsel im Patienten gezielt in eine beliebige Richtung bewegt werden. Einsatzbereiche sind vor allem Hohlorgane, insbesondere z.B. der menschliche Gastrointestinaltrakt, der mit der Kapsel in einem einzigen Durchgang in seiner Gesamtheit durchfahrbar ist.

Das oben genannte Gesamtsystem bzw. Untersuchungsverfahren wird auch MGCE (magnetically guided capsule endoscopy) genannt. Bei einer ersten Generation dieser Geräte bzw. Kapseln ist der jeweilige räumliche Aufenthaltsort der Kapsel im bzw. relativ zum Spulensystem nicht bekannt, da das System nicht über ein teures und aufwändiges Ortungssystem verfügt. Der Benutzer, welcher die Kapsel durch Bedienung der Anlage händisch steuert, kann diese daher von außen nicht sehen bzw. orten.

Prinzipiell könnte eine derartige Kapsel im Patienten mit Hilfe von Röntgen bzw. Fluoroskopie leicht sichtbar gemacht werden, jedoch stehen dem.die Röntgenbelastung des Patienten und die Kosten für eine dementsprechende Systemerweiterung entgegen. Vielmehr muss sich der Benutzer rein anhand der von der Kapsel bzw. einem zusätzlich in den Patienten eingeführten Endoskop gelieferten Bilder orientieren, um die Kapsel in eine gewünschte Richtung bzw. an einen gewünschten Ort im Patienten steuern zu können.

Lediglich der aktuelle Raumwinkel bzw. die Orientierung der Längsachse der Kapsel im Raum bzw. Spulensystem ist von außen, also vom Spulensystem her einstellbar. Die gewünschte Richtung wird nämlich durch ein homogenes Magnetfeld appliziert. Die Kapsel richtet sich mit ihrer Mittellängsachse entsprechend diesem homogenen Magnetfeld aus. Jedoch ist bei einer parallelen Ausrichtung von magnetischem Moment der Kapsel und äußerem Magnetfeld die. Kraft auf die Kapsel aufgrund des entsprechenden Kreuzproduktes Null. Folglich dauert es eine gewisse Zeit, bis sich die Kapsel entsprechend in diese Sollrichtung des homogenen Magnetfeldes ausrichtet. Da die Kapsel außerdem z.B. im Magen in Flüssigkeit schwimmt, kann z.B. durch Schwappen der Flüssigkeit, etwa in Folge von Peristaltik, Herzschlag oder Atembewegungen ein Schwingen der Kapsel bzw. ein zeitliches Abweichen von der Soll-Raumrichtung erfolgen, da sie nur von kleinen Kräften in dieser Richtung gehalten wird.

Aus der DE 10 2005 032 577 A1 ist ein Verfahren zur Positionsbestimmung einer in einem Magnetfeld navigierbaren Endoskopiekapsel bekannt, bei dem die von der Endoskopiekapsel aufgenommenen Bilder ausgewertet und zur Steuerung des Magnetfeldes herangezogen werden.

Aufgabe der Erfindung ist es, eine verbesserte Vorrichtung zur Navigation einer Endoskopiekapsel in einem Patienten anzugeben.

Die Aufgabe wird gelöst durch eine Vorrichtung gemäß Patentanspruch 1.

Die Erfindung beruht auf der grundlegenden Erkenntnis, dass Ziel bzw. Sinn und Zweck einer Navigation der Kapsel stets die Annäherung der Kapsel an eine markante Auffälligkeit im Patienten gewünscht ist, die z.B. untersucht oder behandelt werden soll. Eine derartige Auffälligkeit ist z.B. eine Läsion, ein Organausgang, eine bestimmte Organstruktur oder ähnliches. Insbesondere soll mit Hilfe der Kapsel bzw. deren Kamera stets eine i.d.R. optisch hoch auflösende Nahaufnahme der Auffälligkeit und auch eine Übersichtsaufnahme selbiger mit deren Umgebung sein. Für letztere Aufnahme ist dann die Kapsel ein Stück vom entsprechenden Objekt zu entfernen. Grundidee der Erfindung ist es daher, die Ausrichtung der Kapsel auf bzw. die Annäherung oder Entfernung von einem Zielobjekt in Form der Auffälligkeit zu automatisieren.

Gemäß der Erfindung wird daher mit der in der Endoskopiekapsel enthaltenen Kamera zunächst ein erstes Bild eines Objektes im Inneren des Patienten aufgenommen. Das Objekt ist z.B. eine Innenwand des Organs des Patienten, in welchem sich die Kapsel gerade befindet, etwa der Magen bzw. dessen Wand. Im Bild wird dann ein wiedererkennbares Strukturmerkmal des Objekts identifizier. Das Strukturmerkmal muss zur Wiedererkennbarkeit also z.B. gegenüber seiner Umgebung Charakteristiken, z.B. Farbunterschiede, Kanten, klare Umgrenzungen, eine charakteristische Form oder ähnliches aufweisen, um es im aktuellen Bild und auch späteren, vom selben Bereich des Objektes angefertigten Bildern wiedererkennen zu können. Idealerweise wird als Strukturmerkmal die o.g. Auffälligkeit im Patienten gewählt, die Ziel der Navigation der Kapsel ist.

Im weiteren Verlauf des Verfahrens werden automatisch fortlaufend Bilder vom Inneren des Patienten aufgenommen. Der zeitliche Abstand zwischen zwei Aufnahmen ist dabei z.B. anhand der Trägheit der Kapselbewegung so gewählt, dass sich die Ausrichtung der Kapsel nicht soweit ändern kann, dass sich das Strukturmerkmal aus dem Abbildungsbereich der Kamera entfernen könnte. Für jedes der neu aufgenommenen Bilder wird dann das Strukturmerkmal automatisch im Bild gesucht.

Die Endoskopiekapsel wird dann so gesteuert, dass die Position des Strukturmerkmals im Bild unverändert bleibt, während der Abbildungsmaßstab gezielt vergrößert oder verkleinert wird. Mit anderen Worten wird hierzu dann die Entfernung der Kapsel zum Objekt hin verkleinert oder vergrößert, so dass das Objekt - eine in der Regel starre Optik der Kamera vorausgesetzt - im Bild kleiner oder größer wird. Die Steuerung wird unabhängig bzw. entgegen bzw. gerade zur Kompensation von Störkräften durchgeführt, die z.B. die Kapsel seitwärts bewegen wollen.

Mit anderen Worten ist durch das Verfahren sichergestellt, dass das Strukturmerkmal möglichst am gleichen relativen Ort im Bild gehalten wird, egal in welchem Abstand oder Betrachtungswinkel sich die Kapsel zum Strukturmerkmal befindet. Die Kapsel bzw. die Kamera bleibt also stets auf das Strukturmerkmal ausgerichtet.

Durch das Verfahren wird dem Benutzer eine halbautomatische Navigation anhand von Bildinhalten, nämlich dem Strukturmerkmal, zur Verfügung gestellt. Die Ausrichtung der Kapsel ist automatisiert, der Benutzer braucht nur noch die Verschiebung der Kapsel gezielt zu kontrollieren. Eine der häufigsten Aufgaben der Kapselnavigation in der Endoskopie, nämlich die Gewinnung von hochaufgelösten lokalen Fotos sowie Übersichtsaufnahmen von Strukturmerkmalen ist damit vereinfacht.

In einer vorteilhaften Ausgestaltung des Verfahrens werden im ersten Bild mindestens zwei vorgebbare, vom Strukturmerkmal zum Rand führende Richtungen gewählt. Diese Richtungen werden nur im ersten Bild einmal festgelegt und für das weitere Verfahren beibehalten. Die Endoskopiekapsel wird dann zusätzlich so gesteuert, dass das Verhältnis der Abbildungsmaßstäbe der folgenden zum ersten Bild in den jeweiligen Richtungen konstant gehalten oder gezielt beeinflusst wird. So wird die Kapsel entweder in einem gewünschten Winkel vor dem Objekt gehalten - mit anderen Worten die Kapsel auf einer Kugelschale um das Objekt am selben Raumwinkel gehalten - , oder der Winkel zum Objekt gezielt beeinflusst - mit anderen Worten die Kapsel auf der Kugelschale um das Objekt bewegt. Die Richtungen werden dabei so gewählt, dass sich bei einer Veränderung der Ausrichtung der Kamera auf das oder einer Entfernungsänderung zum Objekt die Abbildungsmaßstäbe zwangsweise ändern.

In einer Variante dieser Ausführungsform werden in den gewählten Richtungen die Abstände zwischen Strukturmerkmal und Rand des Bildes automatisch ermittelt. Nun wird ein erstes Verhältnis der gemessenen Abstände zueinander ermittelt. Im Falle mehrerer Abstände sind dies mehrere Verhältnisse. Die Richtungen werden dabei so gewählt, dass sich bei einer Veränderung der Ausrichtung der Kamera auf das oder der Entfernungsänderung zum Objekt die oben ermittelten Verhältnisse der Abstände zwangsweise ändern. Die Abstände in den festgelegten vorgegebenen Richtungen und deren aktuelles Verhältnis zueinander werden dann auch in den folgenden Bildern automatisch ermittelt.

Bei einem Abweichen des aktuellen vom ersten Verhältnis wird dann die Position der Endoskopiekapsel automatisch korrigiert. Die Korrektur erfolgt in eine geeignete Richtung, so dass sich das zu erwartende Verhältnis der Abstände im nächsten aufzunehmenden Bild wieder dem ersten Verhältnis annähert. Mit anderen Worten wird also erfindungsgemäß vorgeschlagen, in aufeinanderfolgend aufgenommenen Bildern die relativen Verkürzungen in unterschiedlichen Richtungen zwischen dem Strukturmerkmal und dem Bildrand auszuwerten und mittels einer Regelschleife die Kapsel so zu steuern, dass diese relativen Verkürzungen konstant bzw. im gleichen Verhältnis gehalten werden.

Wie oben erwähnt, wird in einer bevorzugten Ausführungsform des Verfahrens als Strukturmerkmal eine Läsion von Interesse im Patienten identifiziert. Wie bereits erwähnt, ist die Beobachtung, Abbildung bzw. Untersuchung von Läsionen eine der vorrangigen Aufgaben bei der Kapselendoskopie.

Für die erstmalige Identifizierung des Strukturmerkmals im ersten aufgenommenen Bild existieren zwei Varianten. In einer ersten Ausgestaltung des Verfahrens wird das Strukturmerkmal von einem Benutzer identifiziert. Hierbei kann die Erfahrung des Benutzers bei der Auswahl des Strukturmerkmals von Vorteil sein, da dieser das Objekt seines Interesses in Form des Strukturmerkmals als relativen Fixpunkt im Bild bewusst auswählt.

In einer alternativen Ausgestaltung des Verfahrens wird das Strukturmerkmal jedoch automatisch gesucht. So kann dem Benutzer unter Zuhilfenahme von Bildbearbeitungs- bzw. Methoden der künstlichen Intelligenz die Identifizierung auffälliger Läsionen abgenommen werden, was eine weitere Erleichterung für den Benutzer bedeutet.

In einer weiteren vorteilhaften Ausgestaltung des Verfahrens wird die Endoskopiekapsel zusätzlich zur oben genannten automatischen Ausrichtung bezüglich des Strukturmerkmals auf dieses zu oder von diesem weg bewegt. So ergibt sich eine halbautomatische, also bezüglich ihrer Richtung stabilisierte, Bewegung auf das Strukturmerkmal zu oder von diesem weg. Die Anfertigung der oben angesprochenen Detail- und Übersichtsaufnahme des Strukturmerkmals wird so besonders erleichtert.

In einer vorteilhaften Ausgestaltung dieser Variante wird die Endoskopiekapsel automatisch bis zum Anstoßen an das Objekt auf dieses bzw. das Strukturmerkmal zu bewegt. Z.B. zum Zweck einer Biopsienahme erfolgt so ein automatisches Anfahren des Strukturmerkmales, insbesondere wenn dieses möglichst in der Mitte des ersten Bildes gelegen hatte.

Insgesamt ergeben sich erfindungsgemäß mit den genannten Ausgestaltungen damit automatisierte Manöver, wie "Zurückfahren ohne Änderung des Blickpunktes", "Annäherung an einen wählbaren Punkt im Blickfeld". Die Bewegungen können entweder automatisch bis zum Anstoßen oder interaktiv vom Benutzer mit Vorwärts- oder Rückwärtsbewegungen durchgeführt werden. Der wählbare Punkt, Blickpunkt etc. ist hierbei durch die Auswahl eines beliebigen, im Bild vorhandenen Strukturmerkmales, das keine pathologische Auffälligkeit des Patienten sein muss, frei festlegbar.

In einer alternativen Ausgestaltung des Verfahrens wird die Kapsel zusätzlich zur o.g. automatischen Ausrichtung in konstantem Abstand, also auf einer Kugelfläche, um das Strukturmerkmal herum bewegt. In dieser Verfahrensvariante wird die Entfernung der Kapsel zum Objekt also konstant gehalten und lediglich der Betrachtungswinkel auf das Objekt verändert. Insbesondere dreidimensionale, aus dem Objekt hervorstehende oder in dieses vertiefte Strukturmerkmale können so besonders einfach von verschiedenen Richtungen aus abgebildet werden. Diese Variante ergibt also ein weiteres automatisches Manöver: "Änderung des Blickwinkels ohne Änderung des Blickpunktes", d.h. das Strukturmerkmal bleibt an der gleichen Relativposition im Bild, z.B. in der Bildmitte und nur die Blickrichtung ändert sich.

Die Vorrichtung umfasst neben der Endoskopiekapsel mit Kamera eine Steuer- und Auswerteeinheit mit einer in dieser implementierten Programm zur Ausführung des oben erläuterten Verfahrens in verschiedenen der genannten Ausgestaltungen.

In einer vorteilhaften Ausgestaltung enthält die Vorrichtung eine vom Benutzer bedienbare Eingabeeinheit. So können auch die o.g. Ausgestaltungen des Verfahrens durchgeführt werden, die eine Benutzereingabe erfordern, z.B. zur Identifizierung des Strukturmerkmals.

Fur eine weitere Beschreibung wird auf die Ausführungsbeispiele der Zeichnungen verwiesen. Es zeigen, jeweils in einer schematischen Prinzipskizze:
Fig. 1 ein MGCE-System im Betrieb,
Fig. 2 ein Ablaufprogramm für die halbautomatische Navigation der Endoskopiekapsel aus Fig. 1.

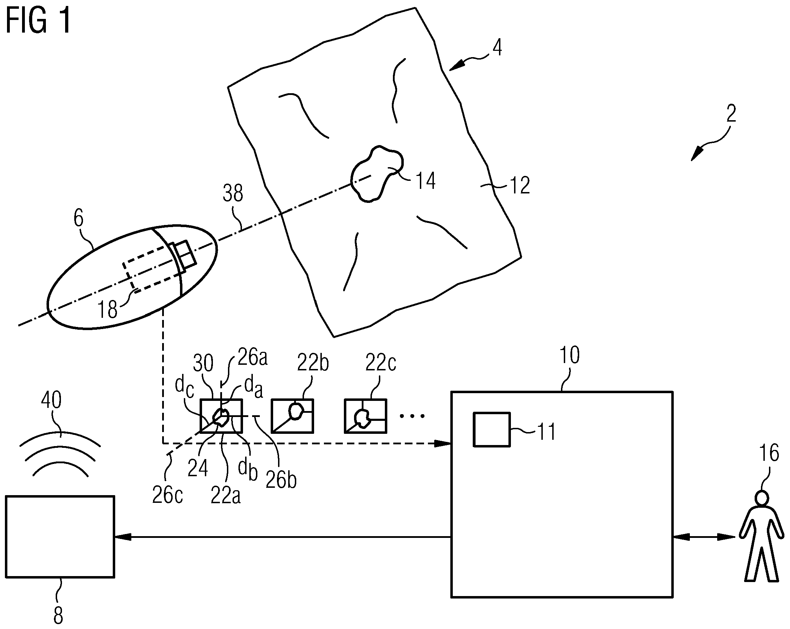

Fig. 1 zeigt ein MGCE-System 2, mit welchem gerade ein Patient 4 untersucht wird. Vom Patienten 4 ist nur ein Objekt 12 in dessen Innerem, nämlich die Magenwand dargestellt. An der Magenwand befindet sich eine Läsion 14. Das MGCE-System 2 umfasst eine in den Patienten 4 eingebrachte Endoskopiekapsel 6, ein Magnetspulensystem 8 zur berührungslosen Kraftausübung auf die Endoskopiekapsel 6, und eine Steuer- und Auswerteeinheit 10 mit einem die unten beschriebenen Abläufe steuernden Programm 11.

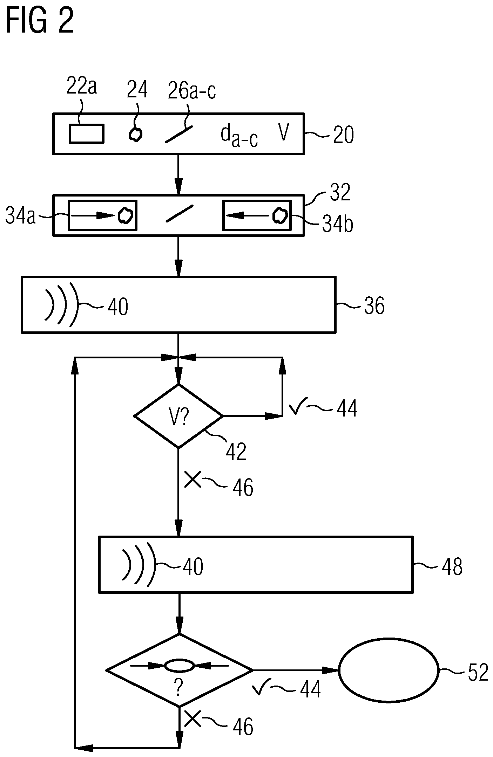

Die Endoskopiekapsel 6 wurde bereits von einem Benutzer 16 per Handsteuerung an einer Eingabeeinheit der Steuer- und Auswerteeinheit 10 in den Magen des Patienten 4 verbracht. Hierzu benutzte der Benutzer 16 von der in der Endoskopiekapsel 6 eingebauten Kamera 18 gelieferte Bilder in Form eines Live-Videobildes. Da der Benutzer 16 die Läsion 14 als für ihn interessante Struktur betrachtet, startet er an der Steuer- und Auswerteeinheit 10 das automatische Verfahren gemäß dem Programm 11, welches in Fig. 2 dargestellt ist.

In einem ersten Schritt 20 nimmt die Kamera 18 ein erstes Bild 22a auf, welches die Läsion 14 abbildet. Im Bild 22a identifiziert der Benutzer 16 die Läsion 14 als Strukturmerkmal 24. In einer alternativen Ausführungsform wird dies vom Programm 11 selbst automatisch mit Hilfe einer Bildverarbeitung durchgeführt.

Drei Richtungen 26a-c werden nun vorgegeben, welche vom Strukturmerkmal 24 zum Rand des Bildes zeigen. Auch dies geschieht wieder gemäß zweier Ausführungsformen durch den Benutzer 16 oder automatisch durch das Programm 11. In den vorgegebenen Richtungen 26a-c werden nun automatisch durch das Programm 11 die jeweiligen Abstände d_{a-c} zwischen Strukturmerkmal 24 und Rand 30 bestimmt. Außerdem werden die ersten Verhältnisse V₁₁=dₐ/d_{b} und V₁₂=d_{b}/d_{c} bestimmt.

In einem nächsten Schritt 32 gibt der Benutzer 16 als Kommando 34a den Befehl "Annäherung an das Strukturmerkmal". In einer alternativen Ausgestaltung des Verfahrens gibt er das Kommando 34b als "Entfernen vom Strukturmerkmal". Die folgenden Ausführungen gelten dann entsprechend.

In einem weiteren Schritt 36 gibt die Steuer- und Auswerteeinheit 10 an das Magnetspulensystem 8 Befehle für die Applikation Feldgradienten in Blickrichtung 38 der Kamera 18, um eine Kraft auf die Endoskopiekapsel 6 auszuüben. Das Magnetspulensystem 8 erzeugt entsprechende Felder 40. Stärke und Richtung der benötigten Felder 40 werden hierbei von der Steuer- und Auswerteeinheit 10 gemäß einer aus der bisherigen Steuerhistorie grob abgeschätzten aktuellen Position und Ausrichtung der Kapsel möglichst genau ermittelt. Bei der Applikation der Felder 40 wird unter anderem auch die Schwerkraft und der aktuelle Auftrieb, welchen die Kapsel erfahrungsgemäß oder bekannter Weise im Patienten 4 erfährt, berücksichtigt.

Durch die Felder 40 erfolgt eine tatsächliche Bewegung der Endoskopiekapsel 6. Die Kamera 18 liefert nun fortlaufend Bilder 22b,c usw. Da die exakte Lage der Endoskopiekapsel 6 im Patienten 4 weder dem Benutzer 16 noch dem MGCE-System 2 bekannt ist, führt die Applikation der zunächst grob abgeschätzten Felder 40 zu einer Bewegung der Kapsel 6 in einer Richtung, welche unter Umständen nicht genau in die gewünschte Richtung - nämlich in Richtung auf die Läsion 14 zu - verläuft. Das Abbild der Läsion 14 wird zwar größer, wandert daher im Bild 22b aber an einen anderen Ort.

In einem Entscheidungsschritt 42 wird daher das Bild 22b automatisch ausgewertet, in dem das Strukturmerkmal 24 in Form der Abbildung der. Läsion 14 erneut automatisch identifiziert bzw. geortet wird. In den nunmehr festgewählten Richtungen 26a-c werden nun die aktuellen Abstände d_{a-c} ermittelt und aus diesen entsprechende aktuelle Verhältnisse V_{21,22} gebildet. Die aktuellen Verhältnisse V_{21,22} werden nun mit den zuvor berechneten ersten Verhältnissen V_{11,12} verglichen. Sind die aktuellen Verhältnisse gleich den ersten Verhältnissen geblieben, erfolgt gemäß einem Ja-Schritt 44 keine Veränderung der Felder 40.

Da im vorliegenden Fall eine Abweichung stattgefunden hat, ermittelt die Steuer- und Auswerteeinheit 10 in einem Nein-Schritt 46 korrigierte Felder 40, um die Endoskopiekapsel 6 genauer in Richtung zur Läsion 14 hin abzulenken. Der Nein-Schritt 46 führt also zur Korrektur von Gradient und Flussrichtung der Felder 40 entgegen der im Bild 22b erkennbaren Abweichung des Strukturmerkmales 24. Mit anderen Worten wird hierdurch die Position der Endoskopiekapsel automatisch in eine Richtung korrigiert, so dass sich das zu erwartende Verhältnis V_{31,32} im nächsten aufzunehmenden Bild 22c dem ersten Verhältnis V_{11,12} wieder annähert.

Gleiches gilt auch für den Fall, dass die Kapsel 6 am aktuellen Ort verbleiben soll, und daher aktuell keine Felder 40 erzeugt werden, jedoch durch Peristaltik des Patienten 4 leicht verdreht wird. Auch dann ändern sich die aktuellen Verhältnisse V_{21,22} und eine entsprechende Korrektur durch Felder 40 wird angestoßen.

In einem Abfrageschritt wird eine Eingabe des Benutzers 16 abgefragt, ob dieser die Bewegung der Endoskopiekapsel 6 im Ja-Schritt 44 beenden möchte, was zum Ende 52 des Verfahrens führt. Wenn nicht, führt ein Nein-Schritt 46 das gewählte Kommando 34a oder 34b weiter, was erneut zum Entscheidungsschritt 42, d.h. der Aufnahme weiterer Bilder 22c usw., Ermittlung der Abstände d_{a,b} usw. führt.

## Patentansprüche

1. Vorrichtung zur Navigation einer Endoskopiekapsel (6) in einem Patienten (4), umfassend eine Endoskopiekapsel (6) mit einer Kamera (18), ein Magnetspulensystem (8) zur berührungslosen Kraftausübung auf die Endoskopiekapsel (6) und eine Steuer- und Auswerteeinheit (10), wobei in der Steuer- und Auswerteeinheit (10) ein Programm (11) zur Durchführung der folgenden Schritte implementiert ist, bei dem:
- mit Hilfe der Kamera (18) ein erstes Bild (22a) eines Objekts (12) im Inneren des Patienten (4) aufgenommen wird, wobei im Bild (22a) ein wieder erkennbares Strukturmerkmal (14) des Objekts (12) identifiziert wird,
- automatisch fortlaufend Bilder (22b,c) vom Inneren des Patienten (4) aufgenommen werden, wobei für jedes Bild (22b, c) :
- die Endoskopiekapsel (6) automatisch so gesteuert wird, dass die Position des Strukturmerkmals (14) im Bild (22b, c) unverändert bleibt, während der Abbildungsmaßstab durch Verkleinerung oder Vergrößerung des Abstandes der Endoskopiekapsel (6) zum Strukturmerkmal (14) vergrößert oder verkleinert wird, **dadurch gekennzeichnet, dass**
- automatisch Abstände (d_{a-c}) zwischen dem Strukturmerkmal (14) und einem Rand (30) des ersten Bildes (22a) in mindestens zwei vorgebbaren, vom Strukturmerkmal (14) zum Rand (30) des Bildes (22a) führenden Richtungen (26a-c) ermittelt werden, und ein erstes Verhältnis (V_{11,12}) der Abstände (d_{a-c}) zueinander ermittelt wird, wobei für jedes Bild der fortlaufenden Bilder:
- das Strukturmerkmal (14) gesucht wird, die aktuellen Abstände (d_{a-c}) in den vorgegebenen Richtungen (26a-c) und deren aktuelles Verhältnis (V₂₁₋₃₂) zueinander ermittelt werden,
- bei Abweichen des aktuellen (V₂₁₋₃₂) vom ersten Verhältnis (V_{11,12}) die Position der Endoskopiekapsel (6) automatisch in eine Richtung korrigiert wird, so dass sich das zu erwartende Verhältnis (V₂₁₋₃₂) im nächsten aufzunehmenden Bild (22b,c) dem ersten Verhältnis (V_{11,12}) annähert.

2. Vorrichtung nach Anspruch 1, **dadurch gekennzeichnet, dass** das auf der Steuer- und Auswerteeinheit (10) der Vorrichtung implementierte Programm dazu ausgebildet ist, das Strukturmerkmal (14) automatisch zu identifizieren.

3. Vorrichtung nach Anspruch 1 oder 2, **gekennzeichnet durch** eine von einem Benutzer (16) bedienbaren Eingabeeinheit (17).

## Claims

1. Device for navigating an endoscopy capsule (6) in a patient (4), comprising an endoscopy capsule (6) with a camera (18), a magnetic coil system (8) for contactlessly exerting force on the endoscopy capsule (6) and a control and evaluation unit (10), wherein
a program (11) for carrying out the following steps is implemented in the control and evaluation unit (10), in which:
- a first image (22a) of an object (12) inside the patient (4) is acquired with the aid of the camera (18), wherein a re-identifiable structure feature (14) of the object (12) is identified in the image (22a),
- images (22b,c) of the inside of the patient (4) are automatically acquired continuously, wherein for each image (22b,c) :
- the endoscopy capsule (6) is controlled automatically, so that the position of the structure feature (14) in the image (22b,c) remains unchanged while the image scale is increased or reduced in size by the distance of the endoscopy capsule (6) relative to the structural feature (14) being increased or reduced in size, **characterised in that**
- distances (d_{a-c}) between the structure feature (14) and an edge (30) of the first image (22a) are automatically determined in at least two predefinable directions (26a-c) leading from the structure feature (14) to the edge (30) of the image (22a), and a first relationship (V_{11,12}) of the distances (d_{a-c}) relative to one another,
wherein for each image of the continuous images:
- the structure feature (14) is sought, the current distances (d_{a-c}) in the predetermined directions (26a-c) are determined and their current relationship (V₂₁₋₃₂) relative to one another is determined,
- if the current relationship (V₂₁₋₃₂) deviates from the first relationship (V_{11,12}), the position of the endoscopy capsule (6) is automatically corrected in one direction, so that the relationship (V₂₁₋₃₂) to be expected in the next image (22b,c) to be acquired approximates the first relationship (V_{11,12}).

2. Device according to claim 1, **characterised in that** the program implemented on the control and evaluation unit (10) of the device is embodied to identify the structure feature (14) automatically.

3. Device according to claim 1 or 2, **characterised by** an input unit (17) operable by a user (16).

## Revendications

1. Dispositif pour la navigation d'une capsule (6) d'endoscopie dans un patient (4), comprenant une capsule (6) d'endoscopie ayant une caméra (18), un système (8) de bobine d'électroaimant pour appliquer sans contact une force à la capsule (6) d'endoscopie et une unité (10) de commande et d'exploitation, dans lequel il est mis en oeuvre, dans l'unité (10) de commande et d'exploitation, un programme (11) pour effectuer les stades suivants :
- à l'aide de la caméra (18), on prend une première image (22a) d'un objet (12) à l'intérieur du patient (4), une caractéristique (14) de structure, reconnaissable à nouveau, de l'objet (12) étant identifiée dans l'image (22a),
- on prend des images (22b, c) qui se suivent automatiquement, de l'intérieur du patient (4), dans lequel, pour chaque image (22b, c) :
- on commande automatiquement la capsule (6) d'endoscopie, de manière à laisser inchangée la position de la caractéristique (14) de structure dans l'image (22b, c) tandis que l'échelle de reproduction est agrandie ou diminuée par agrandissement ou diminution de la distance de la capsule (6) d'endoscopie à la caractéristique (14) de structure, **caractérisé en ce que**
- on détermine automatiquement des distances (d_{a-c}) entre la caractéristique (14) de structure et un bord (30) de la première image (22a) dans au moins deux directions (26a-c) pouvant être prescrites et allant de la caractéristique (14) de structure au bord (30) de l'image (22a), et on détermine un premier rapport (V_{11, 12}) des distances (d_{a-c}) entre elles, dans lequel, pour chacune des images qui se suivent :
- on recherche la caractéristique (14) de structure, on détermine les distances (d_{a-c}) instantanées dans les directions (26a-c) données à l'avance et leur rapport (V₂₁₋₃₂) instantané entre elles,
- s'il y a un écart du rapport (V₂₁₋₃₂) instantané au premier rapport (V_{11, 12}), on corrige la position de la capsule (6) d'endoscopie automatiquement dans une direction, de manière à ce que le rapport (V₂₁₋₃₂) escompté dans l'image (22b, c) prise immédiatement ensuite se rapproche du premier rapport (V_{11, 12}).

2. Dispositif suivant la revendication 1, **caractérisé en ce que** le programme mis en oeuvre sur l'unité (10) de commande et d'exploitation du dispositif est constitué pour identifier automatiquement la caractéristique (14) de structure.

3. Dispositif suivant la revendication 1 ou 2, **caractérisé en ce qu'**une unité (17) d'entrée peut être mise en oeuvre par un utilisateur (16).
